# EUROPEAN PATENT SPECIFICATION

(11) **EP 1 989 544 B1**
(45) Date of publication and mention of the grant of the patent: **22.06.2011**
(21) Application number: 07712817.1
(22) Date of filing: 02.03.2007
(51) Int. Cl.: G01N 33/50

(54) **T CELL ASSAYS**
T-ZELLEN-TESTVERFAHREN
DOSAGES DE LYMPHOCYTES T

(30) Priority: 02.03.2006 GB 0604170; 29.09.2006 GB 0619374; 11.10.2006 GB 0620123
(43) Date of publication of application: 12.11.2008
(73) Proprietor: Antitope Limited, Babraham, Cambridge CB2 4AT (GB)
(72) Inventor: BAKER, Mathew, Cambridge CB2 4AT (GB); CARR, Francis, J., Aberdeen AB23 8WU (GB); RUST, Alyson, Suffolk CB8 0GR (GB); DAVIES, Laura, Cambridge CB3 6HW (GB)
(74) Representative: Chapman, Paul William
(86) International application number: PCT/GB2007/000736
(87) International publication number: WO 2007/099341

(56) References cited:
- EP-A- 1 241 249
- WO-A-97/39721
- WO-A-99/19484
- WO-A-2004/050706

## Description

The present invention relates to novel T cell assay methods, in particular where T cell responses to test antigens are increased by removal of regulatory T cells. The present invention also relates to novel assays where the timing of incubation with antigens or other samples is varied in order to optimize detection of T cell responses. In particular, the invention relates to T cell assays with proteinaceous samples where optimal detection of T cell epitopes is achieved using multiple timepoint measurements of T cell proliferation or cytokine release. In addition, the invention relates to T cell assays where the timing of incubation with antigen with either antigen-presenting cells (APCs) or T cells or both APCs and T cells is varied in order to optimize detection of T cell epitopes. The invention particularly relates to T cell assays with immunomodulatory or toxic samples which directly affect either APCs, T cells or both APCs and T cells. The invention has particular application for measurement of human T cell responses to pharmaceuticals, allergens, irritants or other substances contacted by man.

T cell assays provide an effective method for measuring T cell responses to antigens and other samples, especially in humans. Such assays are considered as *"ex* vivo" assays where blood samples are taken from donors and processed such that primary cultures of blood cells are used directly in such assays. For peptides and proteinaceous samples, *ex vivo* human T cell assays have been used to detect human T cell epitopes for several purposes including evaluating the potential immunogenicity of such samples in man (Jones et al., J. Interferon Cytokine Res., vol 24 (2004) p560-572), defining T cell epitopes within a protein sequence for subsequent inclusion in vaccines, and defining T cell epitopes within a protein sequence for subsequent removal in order to avoid immunogenicity (Jones et al., J. Interferon Cytokine Res., vol 24 (2004) p560-572, and Jones et al., J. Thromb. Haemost., vol 3 (2005) pl-10). Current T cell assay methods broadly involve either incubating peptide or proteinaceous samples with a mixture of APCs and T cells prior to measurement of T cell responses, or incubating peptide or proteinaceous samples with APCs and then adding T cells prior to measurement of T cell responses. In both types of assay, multiple blood samples are used individually for parallel testing of each individual peptide or proteinaceous sample, and T cell responses are then measured usually at a single time point. T cell responses are typically measured either by incorporation of a pulse of radioactive label such as tritiated thymidine (3HTdR) into proliferating T cells ("T cell proliferation") or by release of cytokines such as IL-2 from activated T cells ("cytokine release").

Current T cell assay methods to detect T cell epitopes are limited by one or both of poor sensitivity and/or by interference due to immunomodulatory or toxic samples which inhibit, stimulate or otherwise modify either APCs, T cells or both APCs and T cells. As such, current T cell assay methods may not detect some or all T cell epitopes in certain peptide and proteinaceous samples and may not be applicable to measurement of T cell responses to immunomodulatory or toxic samples including peptide and proteinaceous samples, non-proteinaceous samples including organic molecules, and formulations of proteinaceous and non-proteinaceous samples where the formulation itself may be immunomodulatory or toxic.

In relation to sensitivity, a primary cause of poor sensitivity in *ex vivo* T cell assays may relate to factors in the assay mixture which reduce T cell responses to test antigens including cell types or factors within the assay culture or by the test antigen or test samples themselves. A further cause of poor sensitivity in *ex vivo* T cell assays may relate to the kinetics of T cell responses to T cell epitopes within peptide or proteinaceous samples whereby individual T cell epitopes may induce T cell responses at different times. For T cell proliferation where a single time point is used, T cell proliferation upon addition of certain samples may, on the one hand, be initially rapid but then decline at the time when a pulse of radioactive label is added such that no significant proliferation response is detected. On the other hand, T cell proliferation upon addition of certain other samples may be initially slow at the time when a pulse of radioactive label is added such that no significant proliferation response is detected even though subsequent proliferation becomes significant. For cytokine release where a single time point is used, cytokine production upon addition of certain samples may, on the one hand, be initially rapid but these cytokines may be subsequently consumed by cells within the assay mixture such that no significant cytokine is detected at the single assay time point. On the other hand, cytokine release may be initially slow such that no significant proliferation response is detected at the single assay time point even though subsequent cytokine release becomes significant. The kinetics of proliferation or cytokine release may be influenced by a range of factors such as allotypic variation in T cell responses between different blood samples, efficiency and kinetics of uptake and processing by APCs, efficiency of proteolysis of peptide or proteinaceous samples within APCs, strength and frequency of T cell epitopes within a peptide or proteinaceous sample, binding affinity of T cell epitopes to specific MHC class II allotypes, efficiency of recognition of peptide-MHC class II complexes by T cell receptors, frequency and concentration of co-stimulatory cell surface molecules, concentrations of co-stimulatory cytokines, stimulation of other cells in the assay mix such as CD8⁺ T cells or suppressor T cells, the presence of memory T cells, and the ability of some samples such as small peptides to directly load onto MHC class II molecules expressed on the surface of APCs.

In relation to T cell assay interference by immunomodulatory or toxic samples, such samples may bind directly or be taken up by APCs, T cells or both APCs and T cells. Such samples can down- or up-regulate the normal immunological function of APCs and/or T cells such that T cell epitopes or T cell responses to samples are not detected. Another cause of T cell assay interference by immunomodulatory samples is through toxicity to APCs, T cells or both APCs and T cells. Other causes of T cell assay interference by immunomodulatory samples include up- or down-regulation of subsets of APCs or T cells such as up-regulation of CD8⁺ T cells or suppressor T cells.

In order to usefully exploit T cell assays for a range of applications especially in relation to human pharmaceuticals, there is a need for more sensitive T cell assays methods for optimal detection of T cell epitopes and also a need for T cell assays which can be used with immunomodulatory or toxic samples.

WO 2004/050706 discloses CCR5 and FoxP3 molecules which can be molecules for depletion or targeting in accordance with the present invention. Similarly, Ep-A-1241249 discloses other such molecules such as CTLA-4 and CD4. WO97/39721 discloses Th2-specific cytokine genes, the activity of whose transcription factors can be modulated according to the present invention to regulate their expression. WO99/19484 discloses certain T cell receptor associated molecules (TRAMS).

The present invention is partly based on the discovery that removal of regulatory T cells from T cell assay mixtures results in substantial increases in helper T cell responses to test antigens. Thus, the present invention provides novel T cell assay methods for optimal detection of T cell epitopes where suppressor T cell are removed from cultures resulting in an increase in T cell responses to test antigens. In addition, the present invention provides novel T cell assay methods for optimal detection of immunogenicity in proteins that modulate T cells and/or APCs, or proteins that have a toxic effect on T cells and/or APC. The present invention can also be applied to the detection of non-proteinaceous compounds that can stimulate T cells either directly through the T cell receptor, or by covalently binding to proteins, or by binding directly to peptides bound by MHC class II molecules, or by binding directly to MHC class II molecules. In particular, the invention provides for methods where regulatory T cells which would normally down-regulate effector T cell responses are removed from cultures prior to measurement of responses to test antigens. In addition, the invention provides for methods with multiple time points of measurement where the time points after incubation with antigens or other samples are optimized for detection of T cell responses.

In the first aspect the present invention provides a method for measuring a helper T cell response to a test substance comprising the follows steps:
(a) isolating antigen-presenting cells (APCs) and T cells from a sample obtained from an organism;
(b) depleting regulatory T cells from the isolated cells;
(c) incubating said APCs and regulatory T cell-depleted cells obtained in (b) with the test substance; and
(d) assaying T cell responses to the test substance.

The APCs and T cells are normally obtained from a blood sample. However, different sources of T cells and/or APCs can be used in the invention including those derived from tonsils, Peyer's Patch, tumours and cell lines. In one preferred embodiment, the method is carried out using human peripheral blood mononuclear cells (PBMCs).

As used herein the term "depletion" means elimination of some of the regulatory T cells. This can be done by physically removing the cells or by inhibiting or modulating the action of the T cells. Thus the activity of the targeted T cells is reduced. Preferably 75%, 80%, 85%, 90%, 95%, 97%, 98%, or 99% of the targetted T cell activity is removed by the depletion process.

It will be understood by those skilled in the art that, as part of the present invention, a range of methods for the depletion or targeting of regulatory T cells might be used as alternatives to the depletion of regulatory T cells by virtue of CD25 ^{hi}. It will also be understood that the present invention will also include methods for modulation of the effects of regulatory T cells in T cell assays. For depletion or targeting, molecules expressed on the surface of regulatory T cells may be used in conjunction with or as alternatives to CD25 for the depletion of these cells. Such molecules may include but not be limited to GITR, CTLA-4, CD103, CC chemokine receptor 4, CD62L and CD45RA and may also include surface-associated cytokines or surface forms of cytokines such as IL-10 and TGFβ. Depletion may be achieved by several methods including binding to specific antibodies to adsorb regulatory T cells onto a solid phase, or to cause the destruction or inhibition of such regulatory T cells, or otherwise to separate regulatory T cells from other T cells for the T cell assays. For modulation, molecules secreted by regulatory T cells may be prevented from such secretion or may be blocked/inhibited/destroyed after secretion. Such molecules may include cytokines such as IL-10, IL-4, IL-5 and TGFβ and such molecules may be blocked using organic or inorganic molecules which bind to such molecules, for example antibodies or soluble receptors, or by inhibitory nucleic acids such as siRNA, antisense oligonucletides, or other nucleic acids delivered into regulatory T cells or induced within such cells. Modulation of regulatory T cell activity may also be achieved by targeting receptors or other surface molecules on regulatory T cells including but not limited to GITR, CTLA-4. CD103, CC chemokine receptor 4, CD62L and CD45RA in such a way as to break the suppressive function of these cells. Such inhibition of function may be achieved, for example, by specific antibodies with an agonist function or which may block ligand-target interactions such that regulatory T cells are not removed but are rendered non-functional. Modulation of regulatory T cell activity may also be achieved by blocking the target receptors of molecules secreted by regulatory T cells or by blocking pathways activated or down-regulated by such secreted molecules. Also for modulation, regulatory T cells may be inhibited directly, for example by blocking of transcription factors such as foxp3 or blocking of other functions or pathways related to regulatory T cells. Such inhibition or blocking may be achieved by organic or inorganic molecules, or by inhibitory nucleic acids such as siRNA, antisense oligonucletides, or other nucleic acids delivered into regulatory T cells or induced within such cells. In all cases where organic, inorganic or nucleic acid molecules are used to inhibit the action of or otherwise modulate regulatory T cells, where such molecules themselves interfere with T cell assays, such molecules will preferably be removed from such assays or modified to a form which will not interfere with such assays. For example, specific antibodies or proteins used to remove molecules secreted by regulatory T cells will either be selectively removed prior to T cell assays or will be used in a specific form which will not interfere with T cell assays. For example, for human T cell assays, a human form of an antibody or protein will be used to avoid T cell responses to the antibody or protein itself.

In the T cell assays of the present invention with test antigens that do not modulate T cells and/or APCs (typically proteins or peptides but also non-proteinaceous compounds) the key steps are as follows;
(1) PBMCs are isolated from human blood samples
(2) Optionally CD8⁺ T cells are removed
(3) CD25^{hi+} T cells are depleted
(4) Cultures are incubated with test antigens at one or more concentrations and tested at one or more time points for T cell proliferation and/or cytokine release

Key steps in the T cell assays of the present invention where the test antigens do modulate T cells and/or APCs are as follows;
(1) PBMCs are isolated from human blood samples
(2) APCs are isolated, typically by adherence to plastic, APCs are induced to differentiate using cytokines and the test antigen is added to the APCs
(3) Autologous PBMCs, processed by prior depletion of CD25^{hi+} T cells and optionally CD8⁺ T cells, are mixed with the APCs
(4) Cultures are incubated with test antigens at one or more concentrations and tested at one or more time points for T cell proliferation and/or cytokine release

When the test substances are peptides or proteinaceous samples or non-proteinaceous samples which are not immunomodulatory or toxic to APCs or T cells, blood can used as a source of CD4⁺ T cell and APCs (in the form of monocytes and dendritic cells). Typically a cohort of donors is selected to best represent the number and frequency of HLA-DR allotypes expressed in the world population or in the population under study. Allotypes expressed in the cohort are typically >80% of those expressed in the population with all major HLA-DR alleles. (individual allotypes with a frequency >5% expressed in the world population) being well represented. Alternatively allotypes expressed in the cohort are chosen to over-represent or to comprise HLA allotypes which are thought to be associated with a particular disease under study. In a preferred embodiment of the present invention, PBMCs are prepared from blood samples by fractionation on density gradients and are then depleted of CD8⁺ T cells and CD25^{hi} T cells such that the remaining PBMC comprise mainly CD4⁺ T cells (∼70%) and APCs (monocytes 10-20% and dendritic cells 1-3%). Such CD8⁺ CD25^{hi} depleted PBMC are established in cell culture and one or more peptides or proteinaceous samples or non-proteinaceous samples are added and the cultures incubated.

Measurement of T cell responses can then either be conducted at one fixed timepoint, or at multiple timepoints. These timepoints can be pre-determined by measuring the kinetics of T cell responses to similar samples or an optimisation substance.

The optimal conditions for an assay can be determined by using an optimisation substance. An "optimisation substance" as used herein is a compound that is known to induce T cell responses, such as individual immunomodulatory/toxic peptides/whole proteins, that are of a size and structure similar to the samples to be tested or with similar properties to the test substance. For peptides or proteinaceous samples or non-proteinaceous samples, one or more peptides (typically 9-40 amino acids in length) or whole proteins or non-proteinaceous compounds of a size and structure similar to the samples to be tested can be used as an optimisation substance. The optimisation substances are assayed and the results used to define the kinetics of typical T cell responses. For example, T cell responses are measured at various time points, most commonly between days 4 and 9 after addition of sample using one or more of a range of different alternative assays. Once the kinetics of T cell responses to the optimisation substance are established, a set of assay time points can be defined for subsequent testing of samples. In this manner, T cell responses to test samples can be assayed at one or more suitable time points. Alternatively, or in addition, two or more concentrations can be used to establish the kinetics of T cell responses to the optimisation substance, and samples can then be tested at these concentrations.

T cells response can be measured using a number of different assays such as T cell proliferation by incorporation of a pulse of 3HTdR (or other radioactive, fluorescent or chemiluminescent compounds taken up by proliferating T cells), release of cytokines such as IL-2 or IFNγ, mRNA transcription changes increased transcription of activation marker mRNA, Ca²⁺ flux, and changes in phenotypic markers especially markers for activated T cells. Typically, for peptides or proteinaceous samples, T cell responses will either be measured by incorporation of a pulse of 3HTdR at days 5, 6, 7 and 8 after addition of the sample or by measurement of cytokine release (especially IL-2) at 8 days after addition of the sample (or by both 3HTdR incorporation and cytokine release measurements). As an alternative, especially for peptides with highly overlapping sequences (for example 15mers from a protein sequence with 12 amino acid overlaps), incorporation of a pulse of 3HTdR and/or measurement of cytokine release at a single timepoint, typically day 7 after addition of the test peptide, can be used. Adjacent overlapping peptides are likely to contain T cell epitopes which together enhance the sensitivity for T cell epitope detection.

When the peptide or proteinaceous samples are immunomodulatory or toxic to APCs or T cells, the sample obtained from the organism is processed and the APCs are separate from the other cells. This is typically carried out by adherence to plastic, and the peptide or proteinaceous sample is then incubated with these APCs. APCs can be incubated with cytokines such as interleukin 4, granulocyte-macrophage colony stimulating factor, tumor necrosis factor alpha and interleukin 1 alpha to induce a mature APC phenotype. Samples in standard T cell assays with pre-fractionated APCs will usually require a sample:APC incubation time of up to 48 hours. Preferably, semi-mature APC are generated by incubation in growth medium containing interleukin 4 and granulocyte-macrophage colony stimulating factor for up to 4 days. Samples including immunomodulatory or toxic samples are then added to the semi-mature APC and incubated for a short time. Depending on the toxicity or immunomodulatory function of the sample, incubation times with semi-mature APC can range from 3 to 10 hours. Following sample:APC incubation, exogenous sample is removed by repeated washing of semi-mature APC. Mature sample pulsed APCs are then generated by incubation with a pro-inflammatory stimulus such as tumour necrosis factor or interleukin 1 or CD40 ligand or lipopolysaccharide. Autologous T cells are added, typically CD4⁺ CD⁸⁻ CD25 ^{hi} depleted T cells prepared from PBMCs as above to the mature sample-pulsed APC. CD4⁺ CD⁸⁻ CD25^{hi} depleted T cells are incubated with mature sample pulsed APCs for a range of further incubation time points. An optimisation substance as described above can be used to establish the kinetics of responses with different APC incubation time points and/or different T cell incubation time points. The results obtained with the optimisation substance can be used to define a set of APC incubation and/or T cell incubation time points for subsequent testing of samples. In this manner, T cell responses to test samples are detected at one or more of the assay time points. Alternatively, or in addition, two or more concentrations can be used to establish the kinetics of T cell responses to the optimisation substance and samples can then be tested at these concentrations.

When the sample to be tested is non-proteinaceous, either of the methods above (i.e. methods for peptide or proteinaceous samples with or without immunomodulatory or toxic properties) can be used depending on whether the non-proteinaceous sample is immunomodulatory or toxic to APCs, T cells or both.

For proteinaceous or non-proteinaceous samples which are immunomodulatory to APCs, T cells or both, an optional additional step is to directly test for the up- or down-regulation of phenotypic markers of, for example, T cell activation or APC differentiation. Typical markers of T cell activation include changes in expression of CD69, CD25, CTLA4, GITR and measurement of intracellular Ca²⁺ flux. Common phenotypic markers used to assess APC differentiation include MHC class II, CD80 and CD86, which are all highly expressed on mature APCs. These additional steps can provide information on the kinetics of T cell responses to test samples which assist in defining the assay timepoints for optimally testing for T cell responses to test samples.

Novel *ex vivo* T cell assay methods of the present invention have a range of applications especially in relation to pharmaceuticals for human use. For proteins for prospective use as pharmaceuticals, T cell assays of the present invention can be used to identify T cell epitopes within the protein sequence by testing overlapping peptides from the protein sequence. The location and strength of such T cell epitopes can then be used for assessment of the potential immunogenicity of the protein in man. Alternatively, T cell epitopes within the protein can be subsequently removed by mutation of the protein sequence prior to use in man. T cell epitopes within certain proteins may also be identified by methods of the present invention and then incorporated into vaccines either by inclusion of the T cell epitope sequence (or variant thereof) within a protein vaccine or for addition to other components as part of a vaccine.

Novel T cell assays of the present invention can be used for assessment of the potential immunogenicity of a range of types of molecules including peptides, proteins and non-proteins including organic molecules, lipids, carbohydrates or molecules composed of two or more different moieties including conjugates, mixtures and formulations. T cell assays of the present invention have broad application in both research, development, manufacture and clinical testing of pharmaceuticals. In research, for example, T cell responses to different analogues of active molecules can be used to assess potential immunogenicity of these analogues in man. Such T cell responses can thus be used as criteria for selection of lead pharmaceuticals for further development. In development, for example, T cell responses to different formulations of the same molecule can be determined to assess potential immunogenicity of these formulations in man. Such T cell responses can thus be used as criteria for selection of the optimal formulation for clinical trials. In manufacture, for example, T cell responses to manufacturing batches of the same molecule can be determined to assess potential immunogenicity of these batches and also to assess any changes in the molecule between batches. Such T cell responses can be used as a quality test for manufacturing. In clinical testing, for example, T cell responses can be determined using patient blood in order, for example, to assess immunogenicity to the pharmaceutical undergoing trials. T cell assays of the present invention could also be used in clinical trials to determine any MHC restriction of T cell responses to the pharmaceutical.

As an alternative to use in detection of T cell epitopes, T cell assay methods of the present invention can be used to assess potential adverse reactions to pharmaceuticals, preferably for human use. These adverse reactions including hypersensitivity, allergy, irritancy, immunosuppression, hyperimmune stimulation and injection site reactions. T cell assay methods of the present invention can be also used to assess potential adverse reactions to non-pharmaceuticals treatments such as transplantation, to environmental agents such as grass pollen allergens, to foodstuffs, to cosmetics, and to a range of industrially produced reagents such as detergents and enzymes.

It will be understood by those skilled in the art that a range of variations in the T cell assay methods of the present invention can be used but that these variations will fall within the scope of the invention, for example by using multiple assay time points in the analysis of T cell responses. For instance, it will be understood that within the scope there are a range of different methods known in the art for analysis of T cell responses including methods such as MHC-peptide binding which determine individual steps towards a T cell response. T cell assays can be performed with APCs enriched for Langerhan cells, different macrophage subsets or different subsets of APCs, and/or using or enriching for different subsets of T cells. It will also be understood that cytokines could be added to (or removed from) the assay mixtures of T cell assays of the present invention in order, for example, to enhance sensitivity or to down- or up-regulate specific APCs or T cells. Different formats of T cell assays can be used in the invention, for example recall assay formats where T cells are primed by APC presentation of a protein or peptide and then re-challenged by the same or a related protein or peptide.

The following examples are provided to illustrate the invention and should not be considered as limiting the scope of the invention.

### Example 1: Effect of CD25⁺ T cell depletion on T cell responses

Peripheral blood mononuclear cells were isolated from healthy community donor buffy coats (from blood drawn within 24 hours) obtained from National Blood Transfusion Service (Addenbrooke's Hospital, Cambridge, UK) and according to approval granted by Addenbrooke's Hospital Local Research Ethics Committee. PBMC were isolated from buffy coats by Ficoll (GE Healthcare, Chalfont St Giles, UK) density centrifugation and CD8+ T cells were depleted using CD8+ RossetteSep^{™} (StemCell Technologies, Vancouver, Canada). Donors were characterized by identifying HLA-DR haplotypes using an Allset^{™} SSP-PCR based tissue-typing kit (Dynal, Wirral, UK) as well as determining T cell responses to a control antigen Keyhole Limpet Haemocyanin (KLH) (Pierce, Cramlington, UK), Tetanus Toxoid (Aventis Pasteur, Lyon, France) and control peptide epitope from Influenza HA (C32, aa 307-319).

CD25 ^{hi} T cell depletion was carried out using anti-CD25 Microbeads from Miltenyi Biotech (Guildford, UK) using the supplier's standard protocol and magnet. 10 vials of each donor was thawed and cells were resuspended in 30mls 2% inactivated human serum/PBS (Autogen Bioclear, Calne, Wiltshire, UK). 5x10⁷ cells were transferred to 3 x 15ml tubes with the remaining cells kept as whole PBMCs. An anti-CD25. microbeads dilution mixture was made using 300µl of beads + 4200µl of separation buffer (0.5% human serum/2mM EDTA/PBS). The 15ml tubes were centrifuged and resuspended in 500µl of microbeads dilution mixture. Tubes were then kept at 4°C for 5, 10 or 20 minutes before separating on the column. Columns were set up by placing column in the magnet supported on a stand, adding 2mls separation buffer to column and allowing it to drip through. After incubation with beads 10ml separation buffer was added and tubes were centrifuged at 1500rpm for 7 minutes. Cells were then resuspended in 500µl of separation buffer and added to the column followed by 2 x 1ml washes with separation buffer. The flow through the column was collected in 15ml tubes and contained the CD25^{hi} T cell depleted fraction. These cells were spun down at 1500rpm for 7 minutes and resuspended in 3ml AIMV medium (Invitrogen, Paisley, UK) before counting.

Cells were stained for CD4 and CD25 and cell numbers detected by FACS. 5-10x10⁵ cells of each cell population were put in one well of a 96-well U bottomed plate (Greiner Bio-One, Frickenhausen, Germany). The plate was spun down at 1200rpm for 4 minutes. Supernatant was ejected and cells were resuspended in 50µl antibody dilution. Antibody dilution consisted of 1/50 dilution of FITC-labelled anti-CD4 antibody (R&D Systems, Minneapolis, USA) + 1/25 dilution of PE-labelled anti-CD25 antibody (R&D Systems, Minneapolis, USA) in FACS buffer (1% human serum/0.01% Sodium azide/PBS). Control wells were also unstained, stained with isotype controls or single stained with labelled antibody.

Plates were incubated on ice for 30 minutes in the dark. Plates were then spun down at 1200rpm for 4 minutes. Supernatant was ejected and cells were resuspended in 200µl FACS buffer. This was repeated twice and cells were then transferred to FACS tubes. Cells were run through a FACS Calibur (Becton Dickinson, Oxford, UK), and data collected and analysed based on size, granularity and fluorescent tags.

Proliferation assays were carried out as follows. Whole CD8⁺ T cell depleted PBMC and CD8⁺ CD25 ^{hi} depleted PBMC were added at 2 x 10⁵ per well in 100µl of AIMV. Using flat bottom 96 well plates, triplicate cultures were established for each test condition. For each peptide 100µl was added to the cell cultures to give a final concentration of 5µM. Cells were incubated with peptides and protein antigens for 7 days before pulsing each well with 1mCi/ml 3HTdR (GE Healthcare, Chalfont St Giles, UK), for 18 hours.

For the proliferation assay, a threshold of a stimulation index equal to or greater than 2 (SI≥2) was used whereby peptides inducing proliferative responses above this threshold were deemed positive (dotted line). All data was analysed to determine the coefficient of variance (CV), standard deviation (SD) and significance (p<0.05) using a one way, unpaired Student's T test. All responses shown with SI≥2 were significantly different (p<0.05)from untreated media controls.

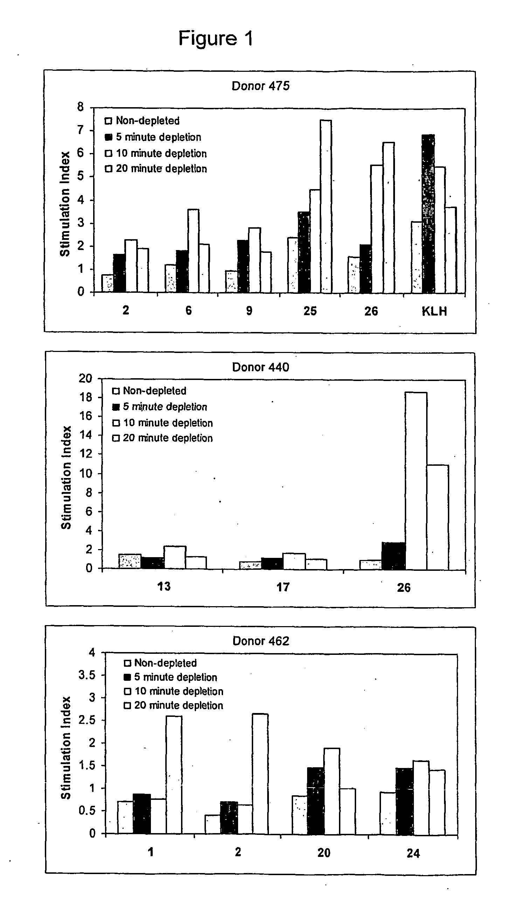

The results are shown in figure 1 which represent T cell proliferative responses in PBMCs from three human donors (475, 440 and 462) to a series of borderline or weak T cell epitopes (peptides 1 (PGQTATITCSGHALG), 2 (GDKFVSWYQQGSGQS), 6 (IKPEAPGCDASPEELNRYYASLRHYLNLVTRQRY), 9 (QSISNWLNWYQQKPG), 13 (KGLEWLVVIWSDGSS), 17 (AASGFTFSSFGMSWV), 20 (DTAVYYCAAAGVRAEDGRVRTLPSEYTFWGQ -GTQV), 24 (HQSLVIKLMPNITLL) and to a pair of strong T cell epitopes (peptides 25 (PKYRNMQPLNSLKIAT) and 26 (TVFYNIPPMPL) and to KLH antigen. The results show an increase in T cell responses for all peptides after depletion of CD25^{hi} T cells. Maximum responses were determined for all peptides following 10 or 20 minute depletion of CD25^{hi} T cells. These results demonstrated strong increases in T cell responses after CD25 ^{hi} T cell depletion which, in the examples of peptides such as peptides 1 and 2, allowed detection of T cell epitopes in peptides previously scored borderline or negative for T cell responses.

### Example 2 - Timecourse Peptide T cell Assays

Wild type (WT) and T cell epitope depleted peptides (HLRHCLSCSKCRKEM and HARHCLSCSKCRKEM, respectively) derived from human sTNFR1 sequence were synthesized (Pepscan Systems, Leystad, Netherlands) and tested using the method of example 1 in which CD8⁺ CD25 ^{hi} T cell depleted PBMC were used to compare peptides derived from sTNFR-1 for the capacity to stimulate T cell responses from twenty healthy donors. Bulk cultures were established by adding 1ml of 2-4x10⁶/ml CD8⁺ CD25 ^{hi} T cell depleted PBMC in AIM V culture medium to each well of a 24 well plate (Greiner Bio-One, Frickenhausen, Germany). Each peptide was tested separately against each donor by adding 1ml 10µM peptide to each bulk culture (final concentration of 5µM for a 2ml per bulk culture). For comparison, additional bulk cultures were established for untreated and positive (KLH) controls. Replicate samples (of T blasts) were removed from bulk cultures on days 6-9 and proliferation was assessed in 96 round bottom plates The data were used to assess the magnitude and kinetics of T cell responses to each peptide on days 6, 7, 8 and 9 post stimulation. In addition, the same twenty healthy donors used in the time course proliferation assay were tested for IL-2 production after 8 days culture with the TNFR1 peptides using the IL-2 Elispot assay. Elispot plates were pre-wet with 70% ethanol then coated with IL-2 capture antibody (R&D systems, Minneapolis, USA) overnight at 4°C. The plates were washed twice with PBS (Invitrogen, Paisley, UK) then blocked in 1% BSA/PBS for 2 hours at room temperature. The plates were washed in PBS prior to the addition of CD8⁺ CD25 ^{hi} T cell depleted PBMC at 4X10⁵ cells per well and test samples at a final concentration of 5µM. After 7 days at 37°C/ 5% CO₂ the plates were developed. After washing with first water then PBS, IL-2 detection antibody (R&D systems, Minneapolis, USA) in PBS/1% BSA was added for 2 hours at 37°C. After further washing with PBS, streptavadin-AP (R&D systems, Minneapolis, USA) was added for 1.5 hours, the plates washed again then BCIP/NBT chromagen (R&D systems, Minneapolis, USA) added for 30 minutes. The plates were washed with water, dried then spot counts analysed using the Immunospot Elispot analyzer, software version 3 (Cleveland, Ohio, USA).

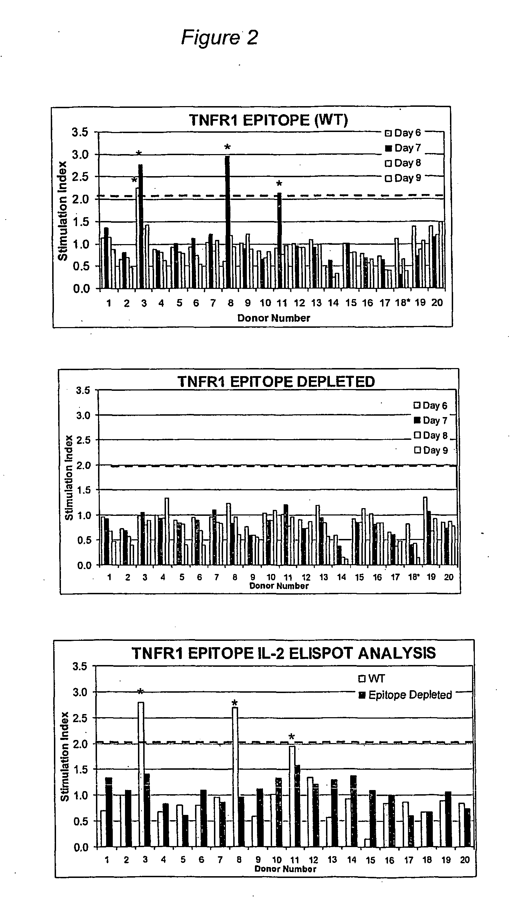

For both T cell proliferation and IL-2 Elispot assays, responses that exceed a SI threshold of 2 (dotted line) and are significantly (p<0.05) different than background (*) were deemed positive. The results shown in figure 2 indicate that the WT peptide gave responses in the same three human donors (3, 8 and 11) in both proliferation and IL-2 Elispot assays indicating that this peptide contains a T cell epitope. The proliferation timecourse indicated that for these three donors, peak proliferation responses were detected 7 days after peptide addition and, in each case, the WT TNFR1 peptide would have been scored negative as a T cell epitope if proliferation responses had been measured at timepoints 8 or 9 days after peptide addition. These results also show a strong correlation between T cell responses measured by proliferation and IL-2 Elispot.

### Example 3 - Timecourse Whole Protein T cell Assays

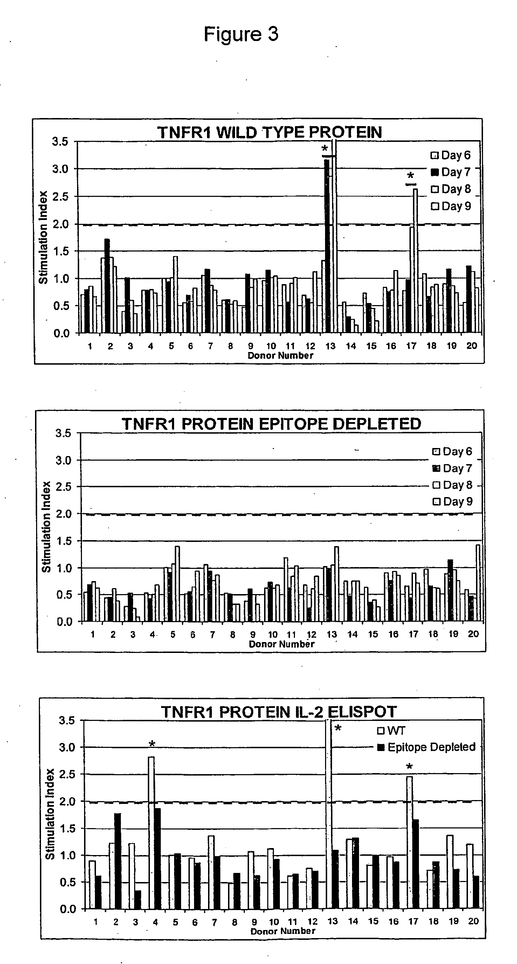

Wild type (WT) and mutant T cell epitope depleted human sTNFR1 proteins were prepared as human Fc fusion proteins as described in WO/2004/113387 with the epitope-depleted protein having mutations I10Q, T20R, H23P, L56A, L108T, L110H and L149D. Proliferation and IL-2 Elispot assays were performed as in example 2 except that 1ml of sTNFR1 proteins were added to a final concentration of 10µg/ml. The data shown in figure 3 indicate that, for the proliferation assay, significant T cell responses were detected in donors 13 and 17 for the WT but not the mutant T cell epitope depleted protein. Peak responses were observed at days 8 and 9 and neither donor 13 or 17 showed any significant response at day 6. For the IL-2 Elispot assay, both donors 13 and 17 were again positive for T cell responses to WT but not the mutant epitope depleted protein. In addition, donor 4 gave a significant response to WT protein in this assay. As with example 2, the results further demonstrate the utility of the timecourse assay in detecting T cell responses, in this case to whole proteins. As with example 2, the results show a good correlation between T cell responses measured by proliferation and IL-2 Elispot.

### Example 4 - Timecourse Immunomodulatory Protein T cell Assays

This example illustrate the invention when used to measure T cell responses to an immunomodulatory protein, human interferon beta which is known to upregulate inhibitory molecules on dendritics cells such as HLA-G (Mitsdoerffer M et al J Neuroimmunol. 2005 159:155-64) and B7-1H (Schreiner B et al J Neuroimmunol. 2004 155:172-82). In order to test whether linear T cell epitopes present in the sequence of IFN beta could stimulate T cells *in vitro,* a modified method for loading antigen into monocyte derived dendritic cells was developed in which the biological effects of IFN beta on both dendritic cells (DC) and CD4⁺ T cells was minimized.

Monocytes were isolated from PBMC by adherence to tissue culture plastic (>90% CD14⁺) and were cultured in 24 well plates in AIM V medium with 5% heat inactivated human AB serum (Autogen Bioclear, Calne, Wiltshire, UK) (growth medium) at an approximate density of 1x10⁶ per well (24 well plate). Monocytes were incubated in growth medium containing human IL-4 (Peprotech, Rocky Hill, NJ, USA) and GM-CSF (Peprotech, Rocky Hill, NJ, USA) for 3 days. On day 3, 44 µg/ml of Betaferon (Schering AG, Berlin, Germany) were added in 0.5ml test buffer plus 3% heat inactivated human AB serum and 25mM (final concentration) HEPES pH 8. Control wells containing 50µg/ml KLH or no antigen (untreated cells) were incubated in 1ml PBS+0.01% Tween 20 plus 3% heat inactivated human AB serum (standard buffer). DC were incubated with antigen for 6 hours after which DCs were washed 6 times to remove exogenous IFN beta. Cells were then resuspended in growth medium containing TNF alpha (Peprotech, Rocky Hill, NJ, USA), GM-CSF and IL-4 overnight.

On day 4, autologous CD8⁺ CD25^{hi} depleted CD4⁺ T cells were isolated by negative selection from PBMC (Dynal Human CD4⁺ Negative Isolation Kit, Wirral, UK) and were then added to DCs at 1x10⁵ per well in both proliferation and Elispot plates. Elispot plates were incubated for 6 days before developing (as in example 2) and proliferation plates were incubated for 7 days before proliferation was measured by incorporation of 3HTdR (a 6 hour pulse at 1 µCi/well).

As with example 2, for proliferation and Elipsot assays an empirical threshold of stimulation index ≥2 was selected where responses above this threshold were deemed positive. Furthermore statistical analysis was also performed to determine whether the responses were significantly (p<0.05) different from untreated control (*). Additional analysis to determine the degree of intra-assay variation included coefficient of variance (CV).

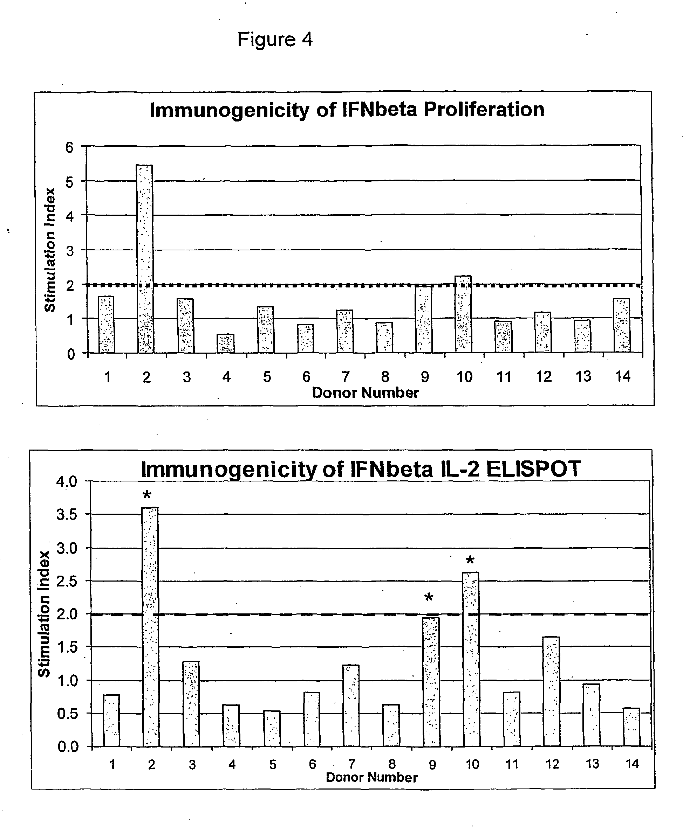

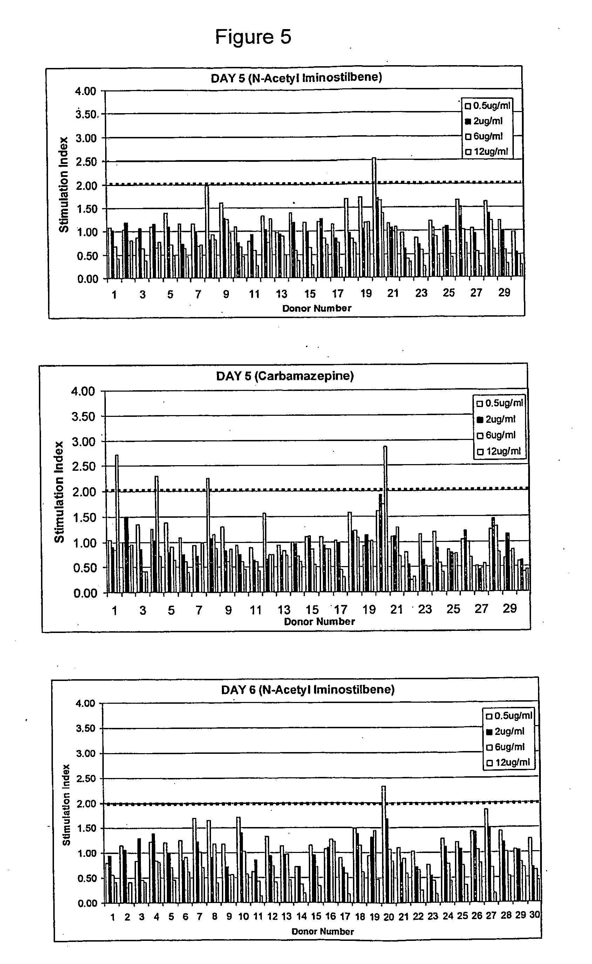

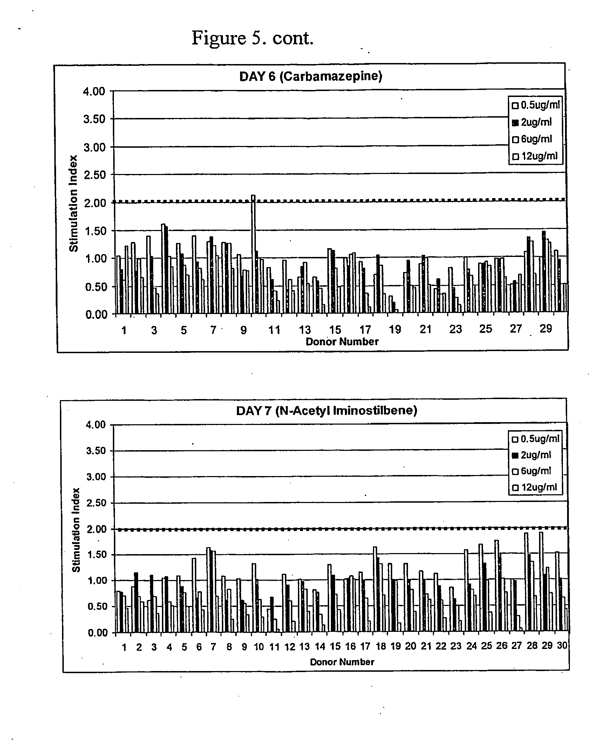

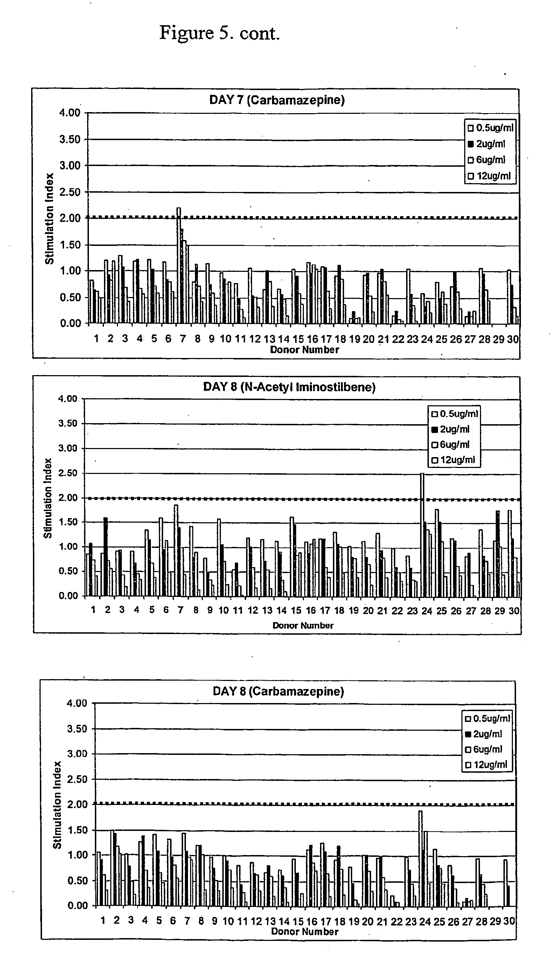

The results, as shown in figure 4, indicate significant T cell responses in 4 out of 29 donors for the proliferation assay and the same 4 out of 29 donors for the IL-2 Elispot assay. This data shows that T cell responses could be reproducibly demonstrated even with an immunomodulatory protein.

### Example 5 - Timecourse Small Molecule T cell Assays

Carbamazepine (Novartis Pharmaceuticals UK) and an N-acetyl iminostilbene (an analogue of carbamazepine, synthesized according to Ying et al Journal of Allergy and Clinical Immunology 2006; 118:233-241) were compared for the ability to stimulate T cell responses in a panel of healthy donors. Both compounds were tested at 25µg/ml in separate bulk cultures for each donor according to the method of example 2. Briefly, bulk cultures were established using 2-4x10⁶ CD8⁺ CD25^{hi} T cell depleted PBMC in each well of a 24 well plate. Replicate samples (of T blasts) are removed from bulk cultures on days 5-8 and proliferation was assessed in 96 well plates. The data were used to assess the magnitude and kinetics of T cell responses to each compound.

As for example 2, a SI≥2 was used as a threshold for positive responses and data was further analyzed to determine the coefficient of variance (CV), standard deviation (SD) and significance (p<0.05) using parametric and non-parametric statistical analysis. Any given compound was considered to be immunogenic only if the response is statistically significant (p<0.05) with an SI≥2.

The results show that the carbamazepine metabolite N-acetyl iminostilbene stimulates fewer donors than carbamazepine (known to be a potent inducer of delayed allergic responses in patients) when tested over a range of concentrations using the time course T cell assay method. It is clear that using a single time point T cell assay in a large number of T cell responses would not have been detected. Indeed the majority of T cell responses against carbamazepine are induced on day 5 with only one additional response detect on days 6 and 7. Assessment of T cell responses against N-acetyl and carbamazepine using a single time point T cell assay on days 6, 7 or 8 would not have discriminated any level of immunogenicity between these two compounds.

## Claims

1. A method for measuring a helper T cell response to a test substance comprising the follows steps:
(a) isolating antigen-presenting cells (APCs) and T cells from a sample obtained from an organism;
(b) depleting regulatory T cells from the isolated cells;
(c) incubating said APCs and regulatory T cell-depleted cells obtained in (b) with the test substance; and
(d) assaying T cell responses to the test substance.

2. The method of claim 1 wherein said method further comprises:
(ai) separating the antigen-presenting cells (APCs) from other cells; and
step (c) comprises incubating the test substance with the separated APCs prior to subsequent addition of regulatory T cell-depleted cells.

3. The method of claim 2 wherein the APCs are treated with cytokines prior to addition of the test substance.

4. The method of claims 1 to 3 wherein the APCs and T cells are derived from peripheral blood mononuclear cells (PBMCs).

5. The method of any one of claims 1 to 4 wherein the APCs and T cells are human.

6. The method of any one of claims 1 to 5 wherein the regulatory T cells are depleted of CD25hi⁺ T cells.

7. The method of any one of claims 1 to 6 wherein the T cells are depleted of CD8+ T cells.

8. The method of any one of claims 1 to 7 wherein the T cell responses are assayed by measuring any one or more of T cell proliferation, cytokine releases, T cell transcription changes, and/ or other markers associated with T cell activation

9. The method of claim 8 where T cell proliferation is measured by uptake of tritiated thymidine.

10. The method of claim 8 where cytokine release is measured by release of IL-2 and/or IFNγ.

11. The method of any one of claims 1 to 10 wherein the T cell responses are assayed at more than one time point during incubation.

12. The method of any one of claims 2 to 11 wherein the APCs are incubated with the test substance for more than one length of time prior to addition of said T cell depleted cells.

13. The method of claim 1-12 wherein the test substance are assayed at a more than one concentrations.

14. The method of any one of claims 1 to 13 wherein an optimisation substance is assayed to determine the optimal time(s) and/or concentrations(s) for assaying the test substance.

15. The method of any one of claims 1 to 13 where the test substance is a protein.

16. The method of any one of claims 1 to 13 where the test substance is a peptide.

17. The method of any one of claims 1 to 13 where the test substance is a non-protein.

18. The method of claim 17 wherein the test substance is an organic molecule, a lipid, a carbohydrate or a molecule composed of two or more moieties including conjugates, mixtures and formulations.

19. The method of any one of claims 1 to 16 where the test substance is immunomodulatory or toxic to T cells and/or APCs.

20. The method of any one of claims 4 to 13 wherein donor PBMCs are used expressing HLA allotypes representing >80% of the expression in the world population or the population under study.

21. The method of any one of claims 4 to 13 wherein donor PBMCs are used to represent specific HLA allotypes linked to a disease under study.

22. The method of any one of claims 1 to 15 where overlapping peptides from a protein sequence are tested in order to identify T cell epitopes in the protein sequence.

23. The method of any one of claims 15 to 18 wherein a series of molecules are tested individually in order to assess relative immunogenicity.

24. The method of claim 23 wherein relative T cell responses are used as a basis to select lead pharmaceuticals for further development.

25. The method of any one of claims 15 to 18 wherein a test substance is analysed in order to assess potential immunogenicity.

26. The method of any one of claims 15 to 18 wherein different formulations of a test substance are analysed in order to assess relative immunogenicity.

27. The method of any one of claims 15 to 18 wherein different manufacturing batches of a test substance are analysed in order to assess potential immunogenicity.

28. The method of of any one of claims 15 to 18 wherein a test substance is analysed using patient blood as a source of T cells in order to assess immunogenicity to the test substance.

29. The use of a method of any one of claims 1 to 15 to identify T cell epitopes in a protein sequence.

30. The use of a method of any one of claims 1 to 18 to assess the immunogenicity of a test substance.

## Patentansprüche

1. Verfahren zum Messen einer T-Helferzellreaktion auf eine Testsubstanz, umfassend die folgenden Schritte:
(a) Isolieren von antigenpräsentierenden Zellen (APCs) und T-Zellen aus einer aus einem Organismus enthaltenen Probe;
(b) Depletieren regulatorischer T-Zellen aus den isolierten Zellen;
(c) Inkubieren der APCs und der in (b) erhaltenen regulatorische-T-Zell-depletierten Zellen mit der Testsubstanz; und
(d) Testen der T-Zellreaktionen auf die Testsubstanz.

2. Verfahren nach Anspruch 1, wobei das Verfahren ferner umfasst:
(ai) Trennen der antigenpräsentierenden Zellen (APCs) von anderen Zellen; und
Schritt (c) umfasst das Inkubieren der Testsubstanz mit den getrennten APCs vor der anschließenden Zugabe von regulatorische-T-Zell-depletierten Zellen.

3. Verfahren nach Anspruch 2, wobei die APCs vor der Zugabe der Testsubstanz mit Zytokinen behandelt werden.

4. Verfahren nach den Ansprüchen 1 bis 3, wobei die APCs und die T-Zellen aus mononukleären Zellen des peripheren Blutes (PBMC-Zellen) abgeleitet werden.

5. Verfahren nach einem der Ansprüche 1 bis 4, wobei es sich bei den APCs und den T-Zellen um menschliche Zellen handelt.

6. Verfahren nach einem der Ansprüche 1 bis 5, wobei die regulatorischen T-Zellen von CD25hi⁺T-Zellen depletiert sind.

7. Verfahren nach einem der Ansprüche 1 bis 6, wobei die regulatorischen T-Zellen von CD8⁺T-Zellen depletiert sind.

8. Verfahren nach einem der Ansprüche 1 bis 7, wobei die T-Zell-Reaktionen getestet werden, indem einer oder mehrere der folgenden Punkte gemessen werden: T-Zellproliferation, Zytokinfreisetzung, T-Zelltranskriptionsänderung und/oder andere mit der T-Zellaktivierung verbundene Marker.

9. Verfahren nach Anspruch 8, wobei die T-Zellproliferation gemessen wird durch Aufnahme von tritiiertem Thymidin.

10. Verfahren nach Anspruch 8, wobei die Zytokinfreisetzung gemessen wird durch die Freisetzung von IL-2 und/oder IFNγ.

11. Verfahren nach einem der Ansprüche 1 bis 10, wobei die T-Zellreaktionen an mehr als einem Zeitpunkt während der Inkubation getestet werden.

12. Verfahren nach einem der Ansprüche 2 bis 11, wobei die APCs vor der Zugabe der T-Zell-depletierten Zellen für mehr als eine Zeitdauer mit der Testsubstanz inkubiert werden.

13. Verfahren nach Anspruch 1-12, wobei die Testsubstanz in mehr als einer Konzentration getestet wird.

14. Verfahren nach einem der Ansprüche 1 bis 13, wobei eine Optimierungssubstanz getestet wird, um die optimale(n) Zeiträume und/oder Konzentrationen zum Testen der Testsubstanz zu bestimmen.

15. Verfahren nach einem der Ansprüche 1 bis 13, wobei die Testsubstanz ein Protein ist.

16. Verfahren nach einem der Ansprüche 1 bis 13, wobei die Testsubstanz ein Peptid ist.

17. Verfahren nach einem der Ansprüche 1 bis 13, wobei die Testsubstanz ein Nicht-Protein ist.

18. Verfahren nach Anspruch 17, wobei die Testsubstanz ein organisches Molekül, ein Lipid, ein Carbohydrat oder ein Molekül ist, das aus zwei oder mehreren Teilen besteht, einschließlich Konjugaten, Gemischen und Formulierungen.

19. Verfahren nach einem der Ansprüche 1 bis 16, wobei die Testsubstanz für T-Zellen und/oder APCs immunmodulatorisch oder toxisch ist.

20. Verfahren nach einem der Ansprüche 4 bis 13, wobei Donor-PBMCs verwendet werden, die HLA-Allotype exprimieren, welche > 80% der Exprimierung in der weltweiten Population oder der untersuchten Population präsentieren.

21. Verfahren nach einem der Ansprüche 4 bis 13, wobei die Donor-PBMCs verwendet werden, um die mit einer zu untersuchenden Krankheit verbundenen HLA-Allotype zu präsentieren.

22. Verfahren nach einem der Ansprüche 1 bis 15, wobei überlappende Peptide aus einer Proteinsequenz getestet werden, um T-Zellepitope in der Proteinsequenz zu identifizieren.

23. Verfahren nach einem der Ansprüche 15 bis 18, wobei eine Reihe von Molekülen einzeln getestet wird, um die relative Immunogenizität zu beurteilen.

24. Verfahren nach Anspruch 23, wobei die relativen T-Zellreaktionen als Grundlage verwendet werden, um die Hauptpharmazeutika für die weitere Entwicklung auszuwählen.

25. Verfahren nach einem der Ansprüche 15 bis 18, wobei eine Testsubstanz analysiert wird, um die potenzielle Immunogenizität zu beurteilen.

26. Verfahren nach einem der Ansprüche 15 bis 18, wobei unterschiedliche Formulierungen einer Testsubstanz analysiert werden, um die relative Immunogenizität zu beurteilen.

27. Verfahren nach einem der Ansprüche 15 bis 18, wobei unterschiedliche Herstellungschargen einer Testsubstanz analysiert werden, um die potenzielle Immunogenizität zu beurteilen.

28. Verfahren nach einem der Ansprüche 15 bis 18, wobei eine Testsubstanz mithilfe von Patientenblut als T-Zellquelle analysiert wird, um die Immunogenizität auf die Testsubstanz zu beurteilen.

29. Verwendung eines Verfahrens nach einem der Ansprüche 1 bis 15, um T-Zellepitope in einer Proteinsequenz zu identifizieren.

30. Verwendung eines Verfahrens nach einem der Ansprüche 1 bis 18, um die Immunogenizität auf eine Testsubstanz zu beurteilen.

## Revendications

1. Procédé de mesure d'une réponse de lymphocytes T auxiliaires à une substance d'essai comprenant les étapes suivantes consistant à :
(a) isoler les cellules comprenant un antigène (APC) et les lymphocytes T d'un échantillon obtenu auprès d'un organisme ;
(b) appauvrir les cellules isolées en lymphocytes T de régulation ;
(c) incuber lesdites cellules appauvries en APC et en lymphocytes T de régulation obtenues dans (b) avec la substance d'essai ; et
(d) doser les réponses des lymphocytes T à la substance d'essai.

2. Procédé selon la revendication 1, dans lequel ledit procédé comprend en outre :
(ai) la séparation des cellules comprenant un antigène (APC) des autres cellules ; et l'étape (c) comprend l'incubation de la substance d'essai avec les APC séparées avant l'addition ultérieure des cellules appauvries en lymphocytes T de régulation.

3. Procédé selon la revendication 2, dans lequel les APC sont traitées avec des cytokines avant l'addition de la substance d'essai.

4. Procédé selon les revendications 1 à 3, dans lequel les APC et les lymphocytes T proviennent de cellules mononucléaires sanguines périphériques (PBMC).

5. Procédé selon l'une quelconque des revendications 1 à 4, dans lequel les APC et les lymphocytes T sont humains.

6. Procédé selon l'une quelconque des revendications 1 à 5, dans lequel les lymphocytes T de régulation sont appauvris en cellules CD25hi⁺.

7. Procédé selon l'une quelconque des revendications 1 à 6, dans lequel les lymphocytes T sont appauvris en cellules CD8+ T.

8. Procédé selon l'une quelconque des revendications 1 à 7, dans lequel les réponses des lymphocytes T sont dosées par mesure de l'une quelconque ou plusieurs des caractéristiques parmi la prolifération des lymphocytes T, la libération de cytokines, les changements de transcription des lymphocytes T et/ou d'autres marqueurs associés à une activation des lymphocytes T.

9. Procédé selon la revendication 8, dans lequel la prolifération des lymphocytes T est mesurée par l'absorption de thymidine tritiée.

10. Procédé selon la revendication 8, dans lequel la libération de cytokines est mesurée par la libération de IL-2 et/ou de IFNγ.

11. Procédé selon l'une quelconque des revendications 1 à 10, dans lequel les réponses des lymphocytes T sont dosées plus d'une fois pendant l'incubation.

12. Procédé selon l'une quelconque des revendications 2 à 11, dans lequel les APC sont incubées avec la substance d'essai pendant plus d'une période de temps avant l'addition desdites cellules appauvries en lymphocytes T.

13. Procédé selon l'une quelconque des revendications 1 à 12, dans lequel la substance d'essai est dosée en plus d'une concentration.

14. Procédé selon l'une quelconque des revendications 1 à 13, dans lequel une substance d'optimisation est dosée pour déterminer le ou les moments optimaux et/ou la ou les concentrations optimales pour doser la substance d'essai.

15. Procédé selon l'une quelconque des revendications 1 à 13, dans lequel la substance d'essai est une protéine.

16. Procédé selon l'une quelconque des revendications 1 à 13, dans lequel la substance d'essai est un peptide.

17. Procédé selon l'une quelconque des revendications 1 à 13, dans lequel la substance d'essai n'est pas une protéine.

18. Procédé selon la revendication 17, dans lequel la substance d'essai est une molécule organique, un lipide, un sucre ou une molécule constituée de deux groupements ou plus notamment des conjugués, des mélanges et des formulations.

19. Procédé selon l'une quelconque des revendications 1 à 16, dans lequel la substance d'essai est immunomodulatrice ou toxique vis-à-vis des lymphocytes T et/ou des APC.

20. Procédé selon l'une quelconque des revendications 4 à 13, dans lequel des PBMC donneuses sont utilisées exprimant des allotypes de HLA représentant > 80 % de l'expression de la population mondiale ou de la population étudiée.

21. Procédé selon l'une quelconque des revendications 4 à 13, dans lequel des PBMC donneuses sont utilisées pour représenter des allotypes de HLA spécifiques liés à une maladie étudiée.

22. Procédé selon l'une quelconque des revendications 1 à 15, dans lequel des peptides chevauchants provenant d'une séquence de protéine sont étudiés afin d'identifier des épitopes de lymphocytes T dans la séquence de protéine.

23. Procédé selon l'une quelconque des revendications 15 à 18, dans lequel une série de molécules est étudiée individuellement afin de déterminer l'immunogénicité relative.

24. Procédé selon la revendication 23, dans lequel les réponses des lymphocytes T sont utilisées comme base pour choisir des produits pharmaceutiques principaux pour un développement plus poussé.

25. Procédé selon l'une quelconque des revendications 15 à 18, dans lequel une substance d'essai est analysée afin de déterminer l'immunogénicité potentielle.

26. Procédé selon l'une quelconque des revendications 15 à 18, dans lequel différentes formulations d'une substance d'essai sont analysées afin de déterminer l'immunogénicité relative.

27. Procédé selon l'une quelconque des revendications 15 à 18, dans lequel différents lots de fabrication d'une substance d'essai sont analysés afin de déterminer l'immunogénicité potentielle.

28. Procédé selon l'une quelconque des revendications 15 à 18, dans lequel une substance d'essai est analysée à l'aide du sang d'un patient comme source de lymphocytes T afin de déterminer l'immunogénicité de la substance d'essai.

29. Utilisation d'un procédé selon l'une quelconque des revendications 1 à 15 pour identifier des épitopes de lymphocytes T dans une séquence de protéine.

30. Utilisation d'un procédé selon l'une quelconque des revendications 1 à 18 pour déterminer l'immunogénicité d'une substance d'essai.
